# EUROPEAN PATENT SPECIFICATION

(11) **EP 1 087 778 B1**
(45) Date of publication and mention of the grant of the patent: **26.10.2005**
(21) Application number: 99927804.7
(22) Date of filing: 29.05.1999
(51) Int. Cl.: A61K 31/70, A61K 38/21, A61K 47/48

(54) **USE OF PEG-IFN-ALPHA AND RIBAVIRIN FOR THE TREATMENT OF CHRONIC HEPATITIS C**
VERWENDUNG VON PEG-IFN-ALPHA UND RIBAVIRIN ZUR BEHANDLUNG CHRONISCHER HEPATITIS C
UTILISATION DE PEG-IFN-ALPHA ET DE RIBAVIRINE POUR TRAITER L'HEPATITE C CHRONIQUE

(30) Priority: 08.06.1998 EP 98110433
(43) Date of publication of application: 04.04.2001
(73) Proprietor: F. HOFFMANN-LA ROCHE AG, 4070 Basel (CH)
(72) Inventor: ZAHM, Friederike, D-79104 Freiburg (DE)
(74) Representative: Kjellsaa-Berger, Hanny
(86) International application number: PCT/EP1999/003746
(87) International publication number: WO 1999/064016

(56) References cited:
- EP-A- 0 510 356
- EP-A- 0 593 868
- EP-A- 0 707 855
- WO-A-95/13090
- WO-A-97/16204
- WO-A-98/48840

## Description

The present invention relates to the field of treatment of chronic hepatitis C infections using an amount of a PEG-IFN-α conjugate in association with Ribavirin effective to treat hepatitis C.

Interferons (IFNs) are naturally occurring proteins which have antiviral, antiproliferative and immunoregulatory activity. Four distinct classes of interferons are known to exist in humans (Pestka et al. (1987) Ann. Rev. Biochem. 56, 727-777 and Emanual & Pestka (1993) J. Biol. Chem. 268, 12565-12569). The IFNα family represents the predominant class of IFNs produced by stimulated peripheral blood leukocytes (Pestka et al., loc. cit.; Havell et al. (1975) Proc. Natl. Acad. Sci. USA 72, 2185-2187; Cavalieri et al. (1977) Proc. Natl. Acad. Sci. USA 74, 3287-3291), and lymphoblastoid and myeloblastoid cell lines (Familletti et al. (1981) Antimicrob. Agents. Chemother. 20, 5-9). The antiviral effect of IFNα is achieved not only by a direct influence on the viruses themselves, but by an activity on their target cells in the sense of a protection against the virus infection. The interferons can exert effects on cancer tumors and can influence the immune system of the body on that, for example, they activate macrophages and NK cells and intensify the expression of various immunologically significant constituents of the cell membrane. Details of the preparation of interferon-cDNA and the direct expression thereof, especially in E. coli, have been the subject of many publications. Thus, for example, the preparation of recombinant interferons is known, for example, from Nature 295 (1982), 503-508, Nature 284 (1980), 326-320, Nature 290 (1981), 20-26, Nucleic Acids Res. 8 (1980), 4057-4074, as well as from European Patents Nos. 32134, 43980 and 211148.

Combination therapy of IFN-α and Ribavirin in the treatment of chronic hepatitis C infections has been proposed (European Patent Application No. 707855), however, this treatment is not always effective.

The combination therapy of PEG-IFN-α conjugates and Ribavirin may thus be more effective than combination therapy of IFN-α and Ribavirin.

It has been observed that in the case of IFN-α, PEGylation increases circulating half-life and plasma residence time, reduces immunogenicity, decreases clearance and increases in vivo activity.

The present invention provides therefore the use of PEG-IFN-α2A conjugate of the formula: 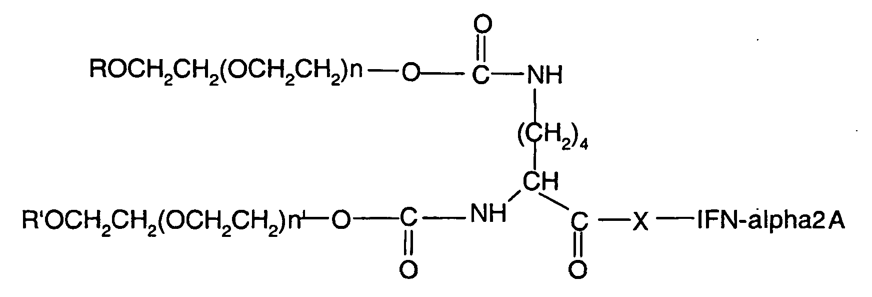 where R and R' are methyl, X is NH, and n and n' are individually or both either 420 or 520 in association with Ribavirin for the manufacture of medicaments for the treatment of chronic hepatitis C infections.

The term "PEG-IFN-α conjugate" as used herein includes IFN-α2As derived from any natural material (e.g., leukocytes, fibroblasts, lymphocytes) or material derived therefrom (e.g. cell lines), or those prepared with recombinant DNA technology. Details of the cloning of IFNα2A and the direct expression thereof, especially in E. coli, have been the subject of many publications. The manufacture of IFNα2A is described in European Patents Nos. 43980 and 211148.

The IFN-α2A is conjugated to polyethylene glycol, to form PEG-IFN-α conjugate of the formula: 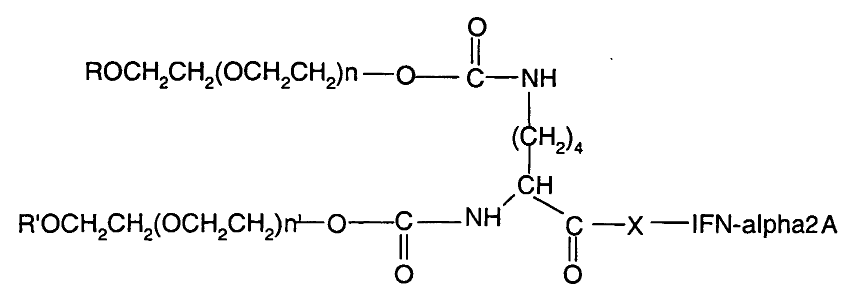 where R and R' are methyl, X is NH, and n and n' are individually or both either 420 or 520, hereinafter referred to as PEG- IFN-α2A conjugate. Conjugation may be accomplished by means of various linkers known in the art, in particularly by linkers such as those disclosed in European Patent Applications, Publication Nos. 0510356, 593868 and 809996.

Ribavirin, 1-β-D-Ribofuranosyl-1H-1,2,4-triazole-3-carboxamide, is described in the Merck Index, compound No. 8199, Eleventh Edition. Its manufacture and formulation are described in U.S. Patent No. 4.211.771.

In accordance with this invention, PEG-IFN-α2A conjugate and Ribavirin are administered to the patient suffering from chronic hepatitis C infection in combined amounts effective to eliminate or at least alleviate one or more of the signs or symptoms of chronic hepatitis C including elevated ALT, positive test for anti-HCV antibodies, presence of HCV as demonstrated by a positive test for HCV-RNA, clinical stigmata of chronic liver disease and hepatocellular damage.

The dosage of PEG-IFN-α2A conjugate for practicing the combination therapy of this invention is about 33 to 540 microgram (mcg) per week, regardless of body weight, in one or two weekly administrations.

The dosage of Ribavirin for practicing this invention is about 400 to 1200 mg per day at least five days per week, preferably seven days per week. Based on the assumption of a patient weighing between 40 and 150 kg, the range of dosing is therefore between 10 and 30 mg per kg body weight per day. In a more specific embodiment the daily dosage of Ribavirin is 800-1200 mg. This daily dosage may be administered once per day in a single dose or in divided doses twice or thrice per day. Preferably the daily dosage of Ribavirin is administered in divided doses twice per day.

In accordance with this invention, the Ribavirin is administered to the patient in association with PEG-IFN-α2A conjugate, that is, the PEG-IFN-α2A conjugate dose is administered during the same or different periods of time that the patient receives doses of Ribavirin. In an embodiment of this invention, at least one daily dose of Ribavirin is administered within the same week as at least one dose of PEG-IFN-α2A. In a more specific embodiment a majority of the Ribavirin administrations occur within the same week as one or more PEG-IFN-α2A administrations. In another specific embodiment, all or substantially all of the Ribavirin administrations occur within the same week as one or more PEG-IFN-α2A administrations. At present PEG-IFN-α2A conjugate formulations are not effective when administered orally, so the preferred method of administering the PEG-IFN-α2A conjugate is parenterally, preferably by subcutaneous (sc) or intramuscular (im) injection. The Ribavirin may be administered orally in capsule or tablet form in association with the parenteral administration of PEG-IFN-α2A conjugate. Of course other types of administration of both medicaments, as they become available are contemplated, such as by nasal spray, transdermally, by suppository, by sustained release dosage form, etc. Any form of administration will work so long as the proper dosages are delivered without destroying the active ingredient.

The effectiveness of treatment may be determined by controlled clinical trials of the combination therapy versus monotherapy and / or combination therapy of IFN-α and Ribavirin. The efficacy of the combination therapy in alleviating the signs and symptoms of chronic hepatitis C infection and the frequency and severity of the side effects will be compared with previous IFN-α monotherapy and / or combination therapy of IFN-α and Ribavirin. Three populations suffering from chronic hepatitis C infection are of relevance for evaluation. Either only one or all three patient populations will be studied with the combination:
1. Patients previously untreated.
2. Patients previously treated with IFN-α and / or Ribavirin or any other drug and who had subsequently relapsed.
3. Patients who were non-responsive to previous treatment with IFN-α and / or Ribavirin or any other drug.

The effectiveness of the combination therapy will be determined by the extent to which the previously described signs and symptoms of chronic hepatitis are alleviated.

### Example

### A Phase III, Randomized, Multicenterr, Efficacy and Safety Study Comparing the Combination of Pegylated-Interferon α2A and Ribavirin to REBETRON™ in the Treatment of Patients with Chronic HCV Infection (CHC).

The primary purpose of this study is to compare the efficacy and safety of the combination of PEG-IFN-α2A and Ribavirin with REBETRON [Intron A + Rebetol (Schering /ICN brand of Ribavirin)] in the treatment of CHC. Equal numbers of patients (330 patients) are receiving either the combination of PEG-IFN-α2A and Ribavirin or REBETRON for 48 weeks. A third group of patients (165 patients) is receiving PEG-IFN-α2A plus placebo for 48 weeks. The monotherapy arm provides a safety and efficacy comparator for the PEG-IFN-α2A combination arm.

The dose of Intron A is 3 Mio. in 0.5 ml solution, administered subcutaneous (sc) three times per week (tiw) for 48 weeks.

The dose of PEG-IFN-α2A is 180 µg, administered sc once per week, in combination with Ribavirin or placebo for 48 weeks.

The dose of Ribavirin and Rebetol is 1000 mg or 1200 mg based upon body weight, per day in split doses. Patients weighing < 75 kg (165 lbs) receive 1000 mg per day (400 mg in the morning and 600 mg in the evening), whereas patients weighing ≥ 75 kg receive 1200 mg per day (600 mg in the morning and 600 mg in the evening).

The primary efficacy parameters are the combined sustained virological [i.e., non-detectable HCV-RNA as measured by the AMPLICOR™ PCR assay (sensitivity ≥ 100 copies/ml)] and biochemical (normalization of serum ALT concentration) responses at the conclusion of the untreated follow-up period. To be considered a responder, patients must have a normal serum alanine aminotransferase (ALT) activity at both weeks 68 and 72 and no detectable virus at week 72.

Safety assessments are performed during screening, at baseline, at weeks 1, 2, 4, 6 and 8 and then every 4 weeks thereafter throughout the 48 week treatment period. Safety assessment continues during the subsequent 24-week follow-up period. Measures of safety include adverse events, vital signs, and laboratory tests as well as tabulations of dose adjustments and premature withdrawals from treatment for safety or tolerability reasons.

Male and female patients aged 18 years or older with CHC who have not previously been treated with any form of IFN-α2A or Ribavirin constitute the patient population. Patients must have quantifiable HCV-RNA, persistently abnormal ALT and liver biopsy within 12 months consistent with CHC. Patients with other forms of liver disease, anemia, human immunodeficiency virus (HIV) infection, hepatocellular carcinoma, pre-existing severe depression or other psychiatric disease, cardiac disease, renal disease, seizure disorders, or severe retinopathy are excluded.

A screening period (time from the first screening assessment to the first administration of test drug) of up to 35 days precedes treatment portion of the trial (48 weeks). Patients meeting all eligibility criteria are randomized to one of the three treatment regimens.

Patients in all groups who do not demonstrate a week 12 response [defined as either a decrease of at least one (1) log 10 unit in their HCV-RNA titer, as compared to baseline, or at least a 50% decrease (or normalization) of their serum ALT, as compared to baseline] are discontinued from therapy and considered non-responders. Patients meeting the week 12 definition of response are discontinued from treatment at week 24 if they do not demonstrate either non-detectable HCV-RNA (<100 copies/ml) or normalization of ALT. Patients discontinued from treatment are followed thereafter only for safety. All patients meeting the weeks 12 and 24 response criteria are treated for 48 weeks. The primary efficacy parameter is the combined virological and biochemical response (HCV-RNA <100 copies/mL and ALT normalization) at the end of the treatment-free follow-up period (24 weeks).

The currently known sustained virological response rates for the combination therapy of Intron A plus Rebetol and estimates of sustained virological response rates for PEG-IFN-α2A monotherapy for 48 weeks (based upon data obtained from the phase II study), and PEG-IFN-α2A plus Ribavirin are summarized below:

| **Known and Estimated Virological Response Rates** | | | | | | | |
|---|---|---|---|---|---|---|---|
| Treatment Group | Treatment Duration | Genotype 1 (A & B) **EOT** | Genotype 1 (A & B) **EOF** | Genotype non-1 **EOT** | Genotype non-1 **EOF** | Pooled **EOT** | Pooled **EOF** |
| N (Proportion of Total) | | 2/3 | | 1/3 | | 1/1 | |
| Intron A | 48 wks | | 9% | | 31% | 29% | 16% |
| **Intron A plus** Rebetol | **48 wks** | | **29%** | | **65%** | **51% 41%** | **41%** |
| PEG-IFN | 48 wks | 60% | *(29%)*^{a} | 70% | *(60%)*^{a} | 62% | *(40%)*^{a} |
| **PEG-IFN plus Ribavirin** | **48 wks** | ***(61%)***^{***a***} | ***(46%)***^{***a***} | ***70%*** | ***(70%)***^{***a***} | ***(66%)***^{***a***} | ***(53%)***^{***a***} |

| | | | | | | | |
|---|---|---|---|---|---|---|---|
| ^{a}: *Percent in parentheses* are response rates estimated based on known response rates shown in the remainder of the table. EOT: End-of-treatment virological response rate (clearance of virus). EOF: End-of-follow-up virological response rate (clearance of virus). | | | | | | | |

## Claims

1. The use of PEG-IFN-α2A conjugate having the formula: 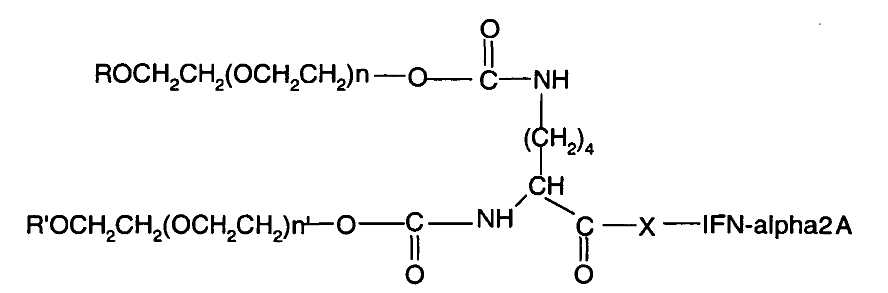 where R and R' are methyl, X is NH, and n and n' are individually or both either 420 or 520 in association with Ribavirin for the manufacture of a medicament for the treatment of chronic hepatitis C infections.

2. PEG-IFN-α2A conjugates having the formula defined in claim 1 in association with ribavirin for the use in therapy.

## Patentansprüche

1. Verwendung eines PEG-IFN-α2A-Konjugats mit der Formel: 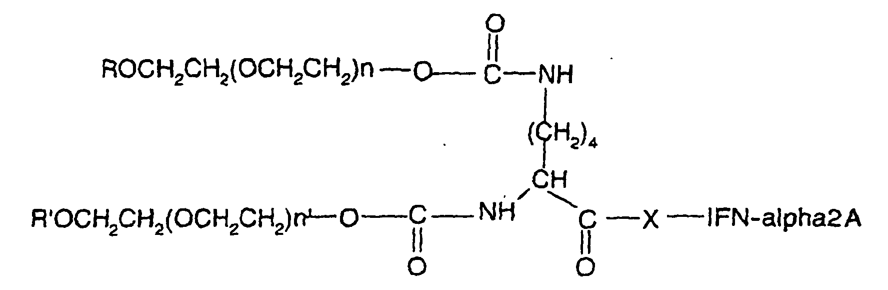 worin R und R' Methyl sind, X NH ist und n und n' jeder für sich oder beide entweder 420 oder 520 sind,
in Verbindung mit Ribavirin zur Herstellung eines Medikaments für die Behandlung von chronischen Hepatitis-C-Infektionen.

2. PEG-IFN-α2A-Konjugate mit der in Anspruch 1 definierten Formel in Verbindung mit Ribavirin für die Verwendung in der Therapie.

## Revendications

1. Utilisation d'un conjugué PEG-IFN-α2A ayant la formule : 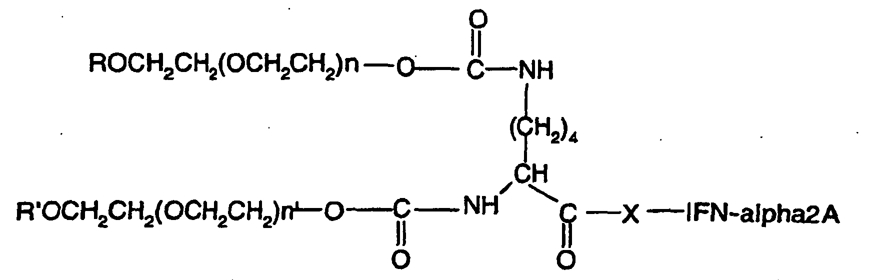 où R et R' sont méthyle, X est NH, et n et n' sont individuellement ou l'un et l'autre 420 ou 520 en association avec la ribavirine pour la production d'un médicament pour le traitement d'infections d'hépatite C chroniques.

2. Conjugués PEG-IFN-α2A ayant la formule définie dans la revendication 1 en association avec la ribavirine destinés à être utilisés en thérapie.
